# EUROPEAN PATENT APPLICATION

(11) **EP 4 011 366 A1**
(43) Date of publication of application: **15.06.2022**
(21) Application number: 20853207.7
(22) Date of filing: 07.08.2020
(51) Int. Cl.: A61K 9/70, A61K 9/00, A61K 47/38, A61K 47/02, A61K 36/185, A61K 31/05

(54) **INTRAORAL FAST-DISINTEGRATING FORMULATION CONTAINING HEMP OIL EXTRACT OR HEMP POWDER EXTRACT AS RAW MATERIAL OF FORMULATION**

(30) Priority: 09.08.2019 KR 20190097649
(71) Applicant: CTC Science Inc., Hwaseong-si, Gyeonggi-do 18576 (KR)
(72) Inventor: JEON, Hong-Ryeol, Suwon-si, Gyeonggi-do 16543 (KR); KWON, Do-Woo, Cheonan-si, Chungcheongbuk-do 31175 (KR); LEE, Bong-Sang, Suwon-si, Gyeonggi-do 16501 (KR); PARK, Su-Jun, Yongin-si, Gyeonggi-do 16823 (KR); KIL, Myeongcheol, Iksan-si, Jeollabuk-do 54664 (KR); YANG, Lee-Seul, Hwaseong-si, Gyeonggi-do 18599 (KR)
(74) Representative: Graf von Stosch Patentanwaltsgesellschaft mbH
(86) International application number: PCT/KR2020/010512
(87) International publication number: WO 2021/029631

(57) **Abstract**

The present disclosure provides an intraoral fast disintegrating formulation containing hemp oil extract or hemp powder extract as a raw material of the formulation, wherein the formulation contains a water-soluble binder, an inorganic material and a surfactant.

## Description

### TECHNICAL FIELD

The present application claims priority to Korean Patent Application No. 10-2019-0097649 filed on August 9, 2019, the disclosures of which are incorporated herein by reference. The present disclosure relates to an intraoral fast disintegrating formulation containing hemp oil extract or hemp powder extract as an active ingredient of the formulation and a method for preparing the same. More specifically, it relates to a fast disintegrating formulation, which contains hemp oil extract or hemp powder extract as an active ingredient and is disintegrated fast in the mouth, and a method for preparing the same.

### BACKGROUND ART

Hemp seed is the seed of hemp also called cannabis or marijuana. With a lower content of tetrahydrocannabinol (THC), which is known as the anesthetic or hallucinogenic ingredient, than hemp cigarette obtained from the leaf or flower of hemp, it is being distributed industrially. Especially, it is drawing attentions as a superfood recently. Rich in high-quality proteins, essential fatty acids, vitamins, minerals, dietary fibers, etc., hemp seed is known to be helpful in preventing cardiovascular diseases and various cancers. It is also known as a good dietary food since it provides satiation. In addition, cannabidiol contained in hemp seed is known to have excellent medicinal effects. Cannabidiol is an ingredient extracted from hemp seed and it is known that it can be used to treat asthma, glaucoma, various cancers, Parkinson's disease, spasm, dementia, arthritis, obesity and anxiety disorder. In addition, it is known to have antibiotic and tension-relieving effects. Although only a little is known about the efficacy and effect of cannabidiol to general consumers in Korea, various products are being marketed globally for treatment of various diseases. However, no product is available and only a few researches are being conducted in Korea.

Hemp extract is being marketed as various oil and powder products for pharmaceuticals and foods globally (typically in Canada, Uruguay and 33 states in the USA). However, hemp oil extract products are inconvenient to carry, have the characteristic unpleasant taste of cannabidiol and are very unfavorable in terms of medication convenience. And, hemp powder products are uneconomical as compared to the hemp oil products because an additional process is required for extraction of the powder. In addition, the characteristic unpleasant taste of cannabidiol remains even after the extraction of the powder from the oil, and it causes inconvenience because it has to be mixed with water or other beverages for intake.

### DISCLOSURE

### Technical Problem

The present disclosure is directed to developing a new formulation, which contains hemp oil extract or hemp powder extract containing a large quantity of cannabidiol as an active ingredient of the formulation and can be disintegrated fast in the mouth, and a method for preparing the same.

### Technical Solution

The present disclosure provides an intraoral fast disintegrating formulation containing hemp oil extract or hemp powder extract as an active ingredient of the formulation, wherein the formulation contains a water-soluble polymer, a water-insoluble excipient which is an inorganic material and a surfactant, and a method for preparing the formulation.

The "hemp oil extract or hemp powder extract" used in the present disclosure is a hemp extract in the form of an oil or a powder. The extract is substantially free from tetrahydrocannabidiol (THC), cannabigerol (CBG), cannabichromene (CBC), cannabinol (CBN), etc., which are known as cannabinoids, and is rich in cannabidiol (CBD). Examples include ORGANIC lisiherb's 80%, 83%, 85% and 90% CBD oils and ORGANIC lisiherb's 99.9% CBD powder. The extraction method for obtaining the extract is not specially limited. For example, any method capable of extracting cannabidiol from hemp, such as distillation, compression, solvent extraction, etc., may be used without limitation.

In an exemplary embodiment, the hemp oil extract contained in the formulation of the present disclosure may contain cannabidiol (CBD) at a content of 80 wt% or more, specifically 83 wt% or more, more specifically 85 wt% or more. And, the hemp powder extract contained in the formulation of the present disclosure contains cannabidiol (CBD) but is substantially free from one or more selected from a group consisting of tetrahydrocannabidiol (THC), cannabigerol (CBG), cannabichromene (CBC) and cannabinol (CBN). The expression 'substantially free from' means that cannabinoid ingredients except cannabidiol (e.g., tetrahydrocannabidiol (THC), cannabigerol (CBG), cannabichromene (CBC) and/or cannabinol (CBN)) are contained at a content of 0.1 wt% or less, 0.07 wt% or less, 0.05 wt% or less, 0.04 wt% or less or 0.03 wt% or less based on the total weight of the hemp powder extract, or they are contained hardly or not at all.

In an exemplary embodiment of the present disclosure, the water-soluble polymer may include one or more selected from a group consisting of hydroxypropyl methylcellulose (HPMC), hydroxypropyl cellulose (HPC), polyvinyl alcohol (PVA), polyvinylpyrrolidone (PVP), carboxymethyl cellulose (CMC), gelatin, pectin, pullulan, polyethylene oxide (PEO) and carbopol. And, the water-soluble excipient may specifically include hydroxypropyl cellulose (HPC), hydroxypropyl methylcellulose (HPMC) or a mixture thereof. The water-soluble excipient may be desirable when considering the compatibility with the active ingredient contained in the formulation, the fast disintegrating property of the formulation and/or the convenience of formulation. Especially, a mixture of hydroxypropyl cellulose (HPC) and hydroxypropyl methylcellulose (HPMC), specifically a mixture of hydroxypropyl cellulose (HPC) and hydroxypropyl methylcellulose (HPMC) at a weight ratio (HPC:HPMC) of 1:1.3-2.5, specifically 1:1.5-2.3, may be used in consideration of film flexibility. The hydroxypropyl cellulose (HPC) may have a viscosity of 2-4000 mPa·S, specifically 10-3500 mPa·S, and the hydroxypropyl methylcellulose (HPMC) may have a viscosity of 2-5.9 mPa·S, specifically 2.5-5 mPa·S.

In an exemplary embodiment of the present disclosure, the water-insoluble excipient which is an inorganic material may include one or more selected from a group consisting of calcium phosphate, calcium sulfate, calcium stearate, calcium carbonate, talc, zinc stearate, titanium dioxide and silicon dioxide, or a hydrate and/or polymorph thereof. Specifically, the water-insoluble excipient may include one or more selected from a group consisting of calcium phosphate, talc, silicon dioxide and titanium dioxide. The use of the inorganic material may be advantageous in terms of improvement of the compatibility with the hemp oil extract or hemp powder extract contained in the formulation, the fast disintegrating property of the formulation and/or the convenience of formulation. Especially, the inorganic material enables fast disintegration of film in the mouth even with a small amount and can significantly reduce foreign body sensation in the mouth. Hereinafter, the "inorganic material" may be understood to refer to the water-insoluble excipient which is an inorganic material.

The inorganic material may be contained at a content of 0.5-3 wt%, specifically 0.7-2.5 wt%, more specifically 1-2 wt%, further more specifically 1.2-1.8 wt%, based on the total weight of the formulation. A fast disintegrating formulation with superior formulation property and little foreign body sensation in the mouth may be provided when the content of the inorganic material is within the above-descried range.

The surfactant contained in the formulation may be a surfactant commonly used in the art to prepare an intraoral fast disintegrating formulation. Specifically, Tween 80, sorbitan ester or a mixture thereof may be used. Specifically, Tween 80 may be used to improve compatibility with the ingredient when considering the purpose of the present disclosure of preparing a formulation by mixing the oil or powder extract with the water-soluble polymer. When a film formulation is prepared by using Tween 80, foaming and lump formation in the prepared film may be reduced significantly. The surfactant may be contained at a content of 1-15 wt%, specifically 3-12 wt%, based on the total weight of the formulation.

The formulation may further contain a thickener, a flavoring agent, and/or a sweetener. The thickener, flavoring agent or sweetener may be any one commonly used in the art. The thickener may include one or more selected from a group consisting of xanthan gum, guar gum and gum arabic, specifically xanthan gum. In addition, the intraoral fast disintegrating film of the present disclosure may further contain a sweetener to cover the bitter taste of cannabidiol in the mouth. The sweetener may be one or more selected from a group consisting of sucralose, neohesperidin, aspartame, stevioside, sorbitol and mannitol. More specifically, two or more sweeteners may be used to cover the bitter taste of cannabidiol. The sweetener may be contained at a content of 1-10 wt%, specifically 3-9 wt%, based on the total dry weight of the film. The flavoring agent may include peppermint oil.

In an exemplary embodiment, when the formulation contains hemp oil extract, the hemp oil extract may be contained at a content of 30-40 wt%, 31-39 wt% or 33-38 wt% based on the total weight of the formulation. When the formulation contains hemp oil extract, the hemp oil extract, the water-soluble polymer and the inorganic material may be mixed at a hemp oil extract : water-soluble polymer : inorganic material weight ratio of 7-12 (specifically 8-10, more specifically 8.5-9.5) : 8-12 (specifically 9-11, more specifically 9.5-10.5) : 0.1-3 (specifically 0.1-2, more specifically 0.2-1).

In an exemplary embodiment, when the formulation contains hemp powder extract, the hemp powder extract may be contained at a content of 25-35 wt%, 27-33 wt% or 28-32 wt% based on the total weight of the formulation. When the formulation contains hemp powder extract, the hemp powder extract, the water-soluble polymer and the inorganic material may be mixed at a hemp powder extract: water-soluble polymer : inorganic material weight ratio of 3-10 (specifically 5-8, more specifically 5.5-7) : 6-15 (specifically 8-11, more specifically 8.5-10.5) : 0.05-1 (specifically 0.05-0.5, more specifically 0.1-0.4).

The inventors of the present disclosure have researched on an optimum content of a formulation containing hemp oil extract or hemp powder extract rich in cannabidiol as an active ingredient of the formulation for improved disintegration rate of the formulation, improved flexibility of the formulation and/or minimized foreign body sensation in the mouth, and have found out that a fast disintegrating formulation having improved flexibility and/or minimized foreign body sensation in the mouth can be provided when the above-described content and/or mixing weight ratio.

In the present disclosure, one or more selected from a group consisting of purified water, ethanol, methanol, DMSO, etc. may be used as a solvent for the intraoral fast disintegrating formulation. Specifically, a combination of purified water and ethanol may be used. In particular, a weight ratio of the purified water and the ethanol may be 1:0.5-2.0.

The intraoral fast disintegrating formulation containing the hemp oil extract or hemp powder extract as an active ingredient may be, for example, an intraoral fast disintegrating film, an intraoral fast disintegrating tablet, etc. It may be any formulation that can be disintegrated fast in the mouth, without being limited thereto. When considering the circumstances where the pharmaceutical formulation is administered as well as portability, medication convenience, etc., the pharmaceutical formulation according to the present disclosure may be specifically an orally dissolving film formulation or a fast disintegrating tablet formulation. Specifically, it may be an orally dissolving film formulation. Specifically, when the formulation is administered orally, 80% or more of the total formulation may be dissolved, dispersed or disintegrated within 2 minutes, specifically within 1 minute and 30 seconds, further more specifically within 1 minute. The term orally dissolving film may be used interchangeably with the terms film, strip, intraoral fast disintegrating film, etc., and may be understood to mean a formulation that can be administered by attaching on the tongue, on the buccal mucosa, under the tongue, etc.

The term "fast disintegration" used in the present disclosure means that 80% or more of the administered film is dissolved, dispersed or disintegrated in the mouth within 2 minutes, specifically within 1 minute, more specifically within 50 seconds, further more specifically 30 seconds. And, the dissolution, dispersion or disintegration of 80% or more of the formulation means that 20 wt% or less of the formulation remains based on its initial weight.

### Advantageous Effects

A formulation of the present disclosure can be disintegrated fast in the mouth.

The formulation of the present disclosure, which contains a large quantity of cannabidiol, can be administered conveniently and can be absorbed quickly into the body.

The formulation containing cannabidiol of the present disclosure has improved bioavailability.

The formulation of the present disclosure contains hemp oil extract or hemp powder extract. The bioavailability of cannabidiol, which is rich in the extract, is decreased significantly to 13-19% as the formulation is absorbed while passing through the gastrointerstinal tract. However, the intraoral fast disintegrating formulation of the present disclosure can provide improved bioavailability because it is disintegrated fast and can be absorbed through the buccal mucosa while bypassing the gastrointerstinal pathway and avoiding hepatic first pass. Slow disintegration may result in insufficient absorption through the buccal mucosa due to slow release from the formulation. In contrast, the formulation of the present disclosure allows fast absorption of cannabidiol through the buccal mucosa owing to fast disintegration.

### MODE FOR DISCLOSURE

Hereinafter, the present disclosure is described in detail through examples. However, the examples of the present disclosure may be changed into various other forms and should not be understood to limit the scope of the present disclosure. The examples of the present disclosure are provided so that the present disclosure can be more fully understood by those having ordinary knowledge in the art.

### <Experimental Example 1. Preparation of film formulation containing hemp oil extract>

A film formulation containing hemp oil extract was prepared as follows. 0.0% THC phytocannabinoid-rich hemp oil (87.06% CBD, 0.0% THC), which is hemp oil extract available from ORGANIC lisiherb, was added to ethanol and then dissolved by stirring. Then, after adding purified water, a surfactant, a flavoring agent, a sweetener, a thickener and other excipients, the mixture was homogenized using a homogenizer (Ultra Turrax T-25, IKA). Then, after adding a water-soluble polymer and an inorganic material, the mixture was homogenized using the same homogenizer. Then, after removing gas from the film solution at 30 °C in vacuo, followed by cooling to room temperature, the solution was coated on a polyethylene terephthalate (PET) film to an appropriate thickness. Then, a film formulation containing hemp oil extract was prepared by drying at 60 °C. The effect of the addition amount of the inorganic material on the disintegration of the film is shown in Tables 1-4.

**[Table 1]**

| Additives | Comp. Ex. 1-1 | Comp. Ex. 1-2 | Ex. 1-1 | Ex. 1-2 | Ex. 1-3 | Comp. Ex. 1-3 | Comp. Ex. 1-4 |
|---|---|---|---|---|---|---|---|
| Hemp oil extract | 9.03 | 9.03 | 9.03 | 9.03 | 9.03 | 9.03 | 9.03 |
| Tween 80 | 1.98 | 1.98 | 1.98 | 1.98 | 1.98 | 1.98 | 1.98 |
| Peppermint oil | 0.94 | 0.94 | 0.94 | 0.94 | 0.94 | 0.94 | 0.94 |
| Sweetener | Adequate | Adequate | Adequate | Adequate | Adequate | Adequate | Adequate |
| TiO₂ | 0.00 | 0.072 | 0.24 | 0.36 | 0.48 | 0.72 | 6.00 |
| HPMC & HPC | 10.06 | 10.06 | 10.06 | 10.06 | 10.06 | 10.06 | 10.06 |
| Purified water & ethanol | To 100% | | | | | | |
| Water-soluble polymer (HPMC & HPC): inorganic material (TiO₂) | - | 139.72:1 | 41.92:1 | 27.94:1 | 20.96:1 | 13.97:1 | 1.68:1 |

| Disintegration | 3 minutes and 30 seconds or longer | About 3 minutes | Within 50 seconds | Within 20 seconds | Within 40 seconds | About 1 minute | About 1 minute |
|---|---|---|---|---|---|---|---|
| Foreign body sensation in mouth | 5 | 5 | 5 | 5 | 5 | 3 | 1 |
| Flexibility | Not broken | Not broken | Not broken | Not broken | Not broken | Not broken | Broken |

**[Table 2]**

| Additives | Comp. Ex. 2-1 | Comp. Ex. 2-2 | Ex. 2-1 | Ex. 2-2 | Ex. 2-3 | Comp. Ex. 2-3 | Comp. Ex. 2-4 |
|---|---|---|---|---|---|---|---|
| Hemp oil extract | 9.03 | 9.03 | 9.03 | 9.03 | 9.03 | 9.03 | 9.03 |
| Tween 80 | 1.98 | 1.98 | 1.98 | 1.98 | 1.98 | 1.98 | 1.98 |
| Peppermint oil | 0.94 | 0.94 | 0.94 | 0.94 | 0.94 | 0.94 | 0.94 |
| Sweetener | Adequate | Adequate | Adequate | Adequate | Adequate | Adequate | Adequate |
| Silicon dioxide | 0.00 | 0.072 | 0.24 | 0.36 | 0.48 | 0.72 | 6.00 |
| HPMC & HPC | 10.06 | 10.06 | 10.06 | 10.06 | 10.06 | 10.06 | 10.06 |
| Purified water & ethanol | To 100% | | | | | | |
| Water-soluble polymer (HPMC & PC): inorganic material (silicon dioxide) | - | 139.72:1 | 41.92: 1 | 27.94:1 | 20.96:1 | 13.97:1 1.68:1 | |
| Disintegration | 4 minutes or longer | About 3 minutes | Within 50 seconds | Within 30 seconds | Within 50 seconds | About 1 minute | About 1 minute |
| Foreign body sensation in mouth | 5 | 5 | 5 | 5 | 5 | 3 | 1 |
| Flexibility | Not broken | Not broken | Not broken | Not broken | Not broken | Not broken | Broken |

**[Table 3]**

| Additives | Comp. Ex. 3-1 | Comp. Ex. 3-2 | Ex. 3-1 | Ex. 3-2 | Ex. 3-3 | Comp. Ex. 3-3 | Comp. Ex. 3-4 |
|---|---|---|---|---|---|---|---|
| Hemp oil extract | 9.03 | 9.03 | 9.03 | 9.03 | 9.03 | 9.03 | 9.03 |
| Tween 80 | 1.98 | 1.98 | 1.98 | 1.98 | 1.98 | 1.98 | 1.98 |
| Peppermint oil | 0.94 | 0.94 | 0.94 | 0.94 | 0.94 | 0.94 | 0.94 |
| Sweetener | Adequate | Adequate | Adequate | Adequate | Adequate | Adequate | Adequate |
| Talc | 0.00 | 0.072 | 0.24 | 0.36 | 0.48 | 0.72 | 6.00 |
| HPMC & HPC | 10.06 | 10.06 | 10.06 | 10.06 | 10.06 | 10.06 | 10.06 |
| Purified water & ethanol | To 100% | | | | | | |
| Water-soluble polymer (HPMC & | - | 139.72:1 | 41.92:1 | 27.94:1 | 20.96:1 | 13.97:1 | 1.68:1 |
| HPC):inorganic material (talc) | | | | | | | |
| Disintegration | 3 minutes and 30 seconds or longer | About 3 minutes | Within 50 seconds | Within 20 seconds | Within 50 seconds | About 1 minute | About 1 minute |
| Foreign body sensation in mouth | 5 | 5 | 5 | 5 | 5 | 3 | 1 |
| Flexibility | Not broken | Not broken | Not broken | Not broken | Not broken | Not broken | Broken |

**[Table 4]**

| Additives | Comp. Ex. 4-1 | Comp. Ex. 4-2 | Ex. 4-1 | Ex. 4-2 | Ex. 4-3 | Comp. Ex. 4-3 | Comp. Ex. 4-4 |
|---|---|---|---|---|---|---|---|
| Cannabidiol oil | 9.03 | 9.03 | 9.03 | 9.03 | 9.03 | 9.03 | 9.03 |
| Tween 80 | 1.98 | 1.98 | 1.98 | 1.98 | 1.98 | 1.98 | 1.98 |
| Peppermint oil | 0.94 | 0.94 | 0.94 | 0.94 | 0.94 | 0.94 | 0.94 |
| Sweetener | Adequate | Adequate | Adequate | Adequate | Adequate | Adequate | Adequate |
| Calcium phosphate | 0.00 | 0.072 | 0.24 | 0.36 | 0.48 | 0.72 | 6.00 |
| HPMC & HPC | 10.06 | 10.06 | 10.06 | 10.06 | 10.06 | 10.06 | 10.06 |
| Purified water & ethanol | To 100% | | | | | | |
| Water-soluble polymer (HPMC & HPC):inorganic material (calcium phosphate) | - | 139.72:1 | 41.92:1 | 27.94:1 | 20.96:1 | 13.97:1 | 1.68:1 |
| Disintegration | 4 minutes or longer | About 3 minutes | Within 40 seconds | Within 20 seconds | Within 40 seconds | About 1 minute | About 1 minute |
| Foreign body sensation in mouth | 5 | 5 | 5 | 5 | 5 | 3 | 1 |
| Flexibility | Not broken | Not broken | Not broken | Not broken | Not broken | Not broken | Broken |

### ① Intraoral disintegration test

The intraoral disintegration rate of the prepared film was tested as follows. The films prepared in Tables 1-4 were cult to a size of 37 x 27 mm and placed in the mouth of 10 adults. Then, the average time spent until the film was disintegrated by saliva was measured (Criterion of evaluation: The film should not be observed in the mouth, and 80% of the entire film should be dissolved, dispersed or disintegrated. The film weight should be 20% or less of the initial weight.).

### ② Intraoral foreign body sensation test

Foreign body sensation in the mouth was tested after the disintegration of the film was completed. It was investigated whether foreign body sensation remains in the mouth after the intraoral disintegration test.

**[Table 5]**

| Score | Evaluation | Standard |
|---|---|---|
| 1 | Poor | Very severe foreign body sensation and unpleasant taste remain in the mouth after disintegration of the film. |
| 2 | Not good | Severe foreign body sensation and unpleasant taste remain in the |
| | | mouth after disintegration of the film. |
| 3 | Not bad | Slight foreign body sensation and unpleasant taste remain in the mouth after disintegration of the film. |
| 4 | Good | No foreign body sensation but slight unpleasant taste remains in the mouth after disintegration of the film. |
| 5 | Excellent | No foreign body sensation or unpleasant taste remains in the mouth after disintegration of the film. |

### ③ Flexibility test

The flexibility of the film was tested. It was investigated whether breakage occurs when the films as described in Tables 1-4 were folded in half (Criterion of evaluation: not broken or broken. Even a slight breakage was evaluated as broken.).

### ④ Test result

The results of testing the intraoral disintegration, foreign body sensation in the mouth and flexibility of the films as described in Tables 1-4 are shown in Table 1-4.

### <Experimental Example 2. Preparation of film formulation containing hemp powder extract>

A film formulation containing hemp powder extract was prepared as follows. ORGANIC lisiherb's CBD powder was added to ethanol and then dissolved by stirring. Then, after adding purified water, a surfactant, a flavoring agent, a sweetener, a thickener and other excipients, the mixture was homogenized using a homogenizer (Ultra Turrax T-25, IKA). Then, after adding a water-soluble polymer and an inorganic material, the mixture was homogenized using the same homogenizer. Then, after removing gas from the film solution at 30 °C in vacuo, followed by cooling to room temperature, the solution was coated on a polyethylene terephthalate (PET) film to an appropriate thickness. Then, a film formulation containing hemp powder extract was prepared by drying at 60 °C. The effect of the addition amount of the inorganic material on the disintegration of the film is shown in Tables 6-9.

**[Table 6]**

| Additives | Comp. Ex. 5-1 | Comp. Ex. 5-2 | Example 5-1 | Example 5-2 | Comp. Ex. 5-3 |
|---|---|---|---|---|---|
| Hemp powder extract | 6.34 | 6.34 | 6.34 | 6.34 | 6.34 |
| Tween 80 | 1.60 | 1.60 | 1.60 | 1.60 | 1.60 |
| Peppermint oil | 1.01 | 1.01 | 1.01 | 1.01 | 1.01 |
| Sweetener | Adequate | Adequate | Adequate | Adequate | Adequate |
| TiO₂ | 0.00 | 0.075 | 0.375 | 0.5 | 6.25 |
| HPMC & HPC | 12.67 | 12.67 | 12.67 | 12.67 | 12.67 |
| Purified water & ethanol | To 100% | | | | |
| Water-soluble polymer (HPMC & HPC):inorganic material (TiO₂) | - | 168.93:1 | 33.79:1 | 25.34:1 | 2.03:1 |
| Disintegration | 4 minutes or longer | About 3 minutes | Within 40 seconds | Within 20 seconds | About 1 minute |
| Foreign body sensation in mouth | 4 | 4 | 5 | 5 | 1 |
| Flexibility | Not broken | Not broken | Not broken | Not broken | Broken |

**[Table 7]**

| Additives | Comp. Ex. 6-1 | Comp. Ex. 6-2 | Example 6-1 | Example 6-2 | Comp. Ex. 6-3 |
|---|---|---|---|---|---|
| Hemp powder extract | 6.34 | 6.34 | 6.34 | 6.34 | 6.34 |
| Tween 80 | 1.60 | 1.60 | 1.60 | 1.60 | 1.60 |
| Peppermint oil | 1.01 | 1.01 | 1.01 | 1.01 | 1.01 |
| Sweetener | Adequate | Adequate | Adequate | Adequate | Adequate |
| Silicon dioxide | 0.00 | 0.075 | 0.375 | 0.5 | 6.25 |
| HPMC & HPC | 12.67 | 12.67 | 12.67 | 12.67 | 12.67 |
| Purified water & ethanol | To 100% | | | | |
| Water-soluble polymer (HPMC & HPC):inorganic material (silicon dioxide) | - | 168.93:1 | 33.79:1 | 25.34: 1 | 2.03:1 |
| Disintegration | 4 minutes or longer | About 3 minutes | Within 40 seconds | Within 20 seconds | About 1 minute |
| Foreign body sensation in mouth | 4 | 5 | 5 | 5 | 1 |
| Flexibility | Not broken | Not broken | Not broken | Not broken | Broken |

**[Table 8]**

| Additives | Comp. Ex. 7-1 | Comp. Ex. 7-2 | Example 7-1 | Example 7-2 | Comp. Ex. 7-3 |
|---|---|---|---|---|---|
| Hemp powder extract | 6.34 | 6.34 | 6.34 | 6.34 | 6.34 |
| Tween 80 | 1.60 | 1.60 | 1.60 | 1.60 | 1.60 |
| Peppermint oil | 1.01 | 1.01 | 1.01 | 1.01 | 1.01 |
| Sweetener | Adequate | Adequate | Adequate | Adequate | Adequate |
| Talc | 0.00 | 0.075 | 0.375 | 0.5 | 6.25 |
| HPMC & HPC | 12.67 | 12.67 | 12.67 | 12.67 | 12.67 |
| Purified water & ethanol | to 100% | | | | |
| Water-soluble polymer (HPMC & HPC):inorganic material (talc) | - | 168.93:1 | 33.79:1 | 25.34:1 | 2.03:1 |
| Disintegration | 4 minutes or longer | About 3 minutes | Within 40 seconds | Within 30 seconds | About 1 minute |
| Foreign body sensation in mouth | 4 | 5 | 5 | 5 | 1 |
| Flexibility | Not broken | Not broken | Not broken | Not broken | Broken |

**[Table 9]**

| Additives | Comp. Ex. 8-1 | Comp. Ex. 8-2 | Example 8-1 | Example 8-2 | Comp. Ex. 8-3 |
|---|---|---|---|---|---|
| Hemp powder extract | 6.34 | 6.34 | 6.34 | 6.34 | 6.34 |
| Tween 80 | 1.60 | 1.60 | 1.60 | 1.60 | 1.60 |
| Peppermint oil | 1.01 | 1.01 | 1.01 | 1.01 | 1.01 |
| Sweetener | Adequate | Adequate | Adequate | Adequate | Adequate |
| Calcium phosphate | 0.00 | 0.075 | 0.375 | 0.5 | 6.25 |
| HPMC & HPC | 12.67 | 12.67 | 12.67 | 12.67 | 12.67 |
| Purified water & ethanol | to 100% | | | | |
| Water-soluble polymer (HPMC & HPC):inorganic material (calcium phosphate) | - | 168.93:1 | 33.79:1 | 25.34:1 | 2.03:1 |
| Disintegration | 4 minutes or longer | About 3 minutes | Within 40 seconds | Within 30 seconds | About 1 minute |
| Foreign body sensation in mouth | 4 | 5 | 5 | 5 | 1 |
| Flexibility | Not broken | Not broken | Not broken | Not broken | Broken |

### ① Intraoral disintegration test

The intraoral disintegration rate of the prepared film was tested as follows. The films as described in Tables 6-9 were cult to a size of 37 x 27 mm and placed in the mouth of 10 adults. Then, the average time spent until the film was disintegrated by saliva was measured (Criterion of evaluation: The film should not be observed in the mouth, and 80% of the entire film should be dissolved, dispersed or disintegrated. The film weight should be 20% or less of the initial weight.).

### ② Intraoral foreign body sensation test

Foreign body sensation in the mouth was tested after the disintegration of the film was completed. It was investigated whether foreign body sensation remains in the mouth after the intraoral disintegration test.

**[Table 10]**

| Score | Evaluation | Standard |
|---|---|---|
| 1 | Poor | Very severe foreign body sensation and unpleasant taste remain in the mouth after disintegration of the film. |
| 2 | Not good | Severe foreign body sensation and unpleasant taste remain in the mouth after disintegration of the film. |
| 3 | Not bad | Slight foreign body sensation and unpleasant taste remain in the mouth after disintegration of the film. |
| 4 | Good | No foreign body sensation but slight unpleasant taste remains in the mouth after disintegration of the film. |
| 5 | Excellent | No foreign body sensation or unpleasant taste remains in the mouth after disintegration of the film. |

**[Table 11]**

| Additives | Comp. Ex. A | Comp. Ex. B | Comp. Ex. C | Comp. Ex. D | Comp. Ex. E | Comp. Ex. F |
|---|---|---|---|---|---|---|
| Hemp extract | 9.03 (hemp oil extract) | 9.03 (hemp oil extract) | 9.03 (hemp oil extract) | 9.03 (hemp powder extract) | 9.03 (hemp powder extract) | 9.03 (hemp powder extract) |
| Tween 80 | 1.98 | 1.98 | 1.98 | 1.98 | 1.98 | 1.98 |
| Peppermint oil | 0.94 | 0.94 | 0.94 | 0.94 | 0.94 | 0.94 |
| Sweetener | Adequate | Adequate | Adequate | Adequate | Adequate | Adequate |
| Water-insoluble excipient (organic material) | 0.36 (chitin) | 0.36 (chitosan) | 0.36 (starch) | 0.375 (chitin) | 0.375 (chitosan) | 0.375 (starch) |
| HPMC & HPC | 10.06 | 10.06 | 10.06 | 10.06 | 10.06 | 10.06 |
| Purified water & ethanol | To 100% | | | | | |

| Disintegration | 3 minutes and 30 seconds or longer | 3 minutes and 30 seconds or longer | 3 minutes and 30 seconds or longer | 3 minutes and 30 seconds or longer | 3 minutes and 30 seconds or longer | 3 minutes and 30 seconds or longer |
|---|---|---|---|---|---|---|
| Foreign body sensation in mouth | 2 | 3 | 1 | 2 | 3 | 1 |
| Flexibility | Broken | Broken | Not broken | Broken | Broken | Not broken |

As can be seen from Table 11, when organic materials of the same amount were added instead of the inorganic material, disintegration time was increased significantly, flexibility was decreased or strong foreign body sensation was felt in the mouth.

Orally disintegrating films with the compositions as described in Tables 12 and 13 were prepared based on the above experimental results.

**[Table 12]**

| Ingredients | Addition amount (mg)/unit | Content (%) | Purpose of addition |
|---|---|---|---|
| Hemp oil extract | 28.72 | 36.80 | Main ingredient |
| Peppermint oil | 3.00 | 3.84 | Flavoring agent |
| Sucralose | 4.00 | 5.12 | Sweetener |
| Neohesperidine | 2.50 | 3.20 | Sweetener |
| Tween 80 | 6.30 | 8.07 | Surfactant |
| TiO₂ | 1.13 | 1.45 | Water-insoluble inorganic material |
| Xanthan gum | 0.40 | 0.51 | Thickener |
| HPMC 2910 5cp | 20.00 | 25.62 | Excipient |
| HPC-L | 12.00 | 15.37 | Excipient |
| Total | 78.05 mg | 100% | |

**[Table 13]**

| Ingredients | Addition amount (mg)/unit | Content (%) | Purpose of addition |
|---|---|---|---|
| Hemp powder extract | 25.00 | 30.37 | Main ingredient |
| Peppermint oil | 3.00 | 3.64 | Flavoring agent |
| Sucralose | 4.00 | 4.86 | Sweetener |
| Neohesperidine | 2.50 | 3.04 | Sweetener |
| Tween 80 | 6.30 | 7.65 | Surfactant |
| TiO₂ | 1.13 | 1.37 | Water-insoluble inorganic material |
| Xanthan gum | 0.40 | 0.49 | Thickener |
| HPMC 2910 5cp | 25.00 | 30.37 | Excipient |
| HPC-L | 15.00 | 18.22 | Excipient |
| Total | 82.33 mg | 100% | |

The film formulations having the composition described in Tables 12 and 13 were disintegrated within 20 seconds in the mouth and showed superior film flexibility with little foreign body sensation after the disintegration.

### INDUSTRIAL APPLICABILITY

The present disclosure provides an intraoral fast disintegrating formulation containing cannabidiol.

The present disclosure also provides a film-type fast disintegrating formulation containing a large quantity of cannabidiol, which is disintegrated fast in the mouth.

## Claims

1. An intraoral fast disintegrating formulation comprising hemp oil extract or hemp powder extract as an active ingredient of the formulation, wherein the formulation is an intraoral fast disintegrating formulation comprising a water-soluble polymer, an inorganic material and a surfactant.

2. The intraoral fast disintegrating formulation according to claim 1, wherein the content of cannabidiol (CBD) in the hemp oil extract is 80% or higher.

3. The intraoral fast disintegrating formulation according to claim 1, wherein the hemp powder extract comprises cannabidiol (CBD) but is substantially free from one or more selected from a group consisting of tetrahydrocannabidiol (THC), cannabigerol (CBG), cannabichromene (CBC) and cannabinol (CBN).

4. The intraoral fast disintegrating formulation according to claim 1, wherein the water-soluble polymer is hydroxypropyl cellulose (HPC), hydroxypropyl methylcellulose (HPMC) or a mixture thereof.

5. The intraoral fast disintegrating formulation according to claim 4, wherein the water-soluble polymer is a mixture of hydroxypropyl cellulose (HPC) and hydroxypropyl methylcellulose (HPMC).

6. The intraoral fast disintegrating formulation according to claims 1, 4 or 5, wherein the water-soluble polymer is a mixture of hydroxypropyl cellulose (HPC) and hydroxypropyl methylcellulose (HPMC) at a weight ratio (HPC:HPMC) of 1:1.3-2.5.

7. The intraoral fast disintegrating formulation according to claim 1, wherein the inorganic material is one or more selected from a group consisting of calcium phosphate, calcium sulfate, calcium stearate, calcium carbonate, talc, zinc stearate, titanium dioxide and silicon dioxide.

8. The intraoral fast disintegrating formulation according to claim 7, wherein the inorganic material is one or more selected from a group consisting of calcium phosphate, talc, silicon dioxide and titanium dioxide.

9. The intraoral fast disintegrating formulation according to claim 1, wherein the surfactant is Tween 80.

10. The intraoral fast disintegrating formulation according to claims 1 or 9, wherein the surfactant is comprised at a content of 1-15 wt% based on the total weight of the formulation.

11. The intraoral fast disintegrating formulation according to any of claims 1 to 10, wherein the formulation further comprises a thickener.

12. The intraoral fast disintegrating formulation according to claim 11, wherein the thickener is one or more selected from a group consisting of xanthan gum, guar gum and gum arabic.

13. The intraoral fast disintegrating formulation according to any of claims 1 to 12, wherein the formulation further comprises a flavoring agent or a sweetener, or further comprises both a flavoring agent and a sweetener.

14. The intraoral fast disintegrating formulation according to any of claims 1 to 13, wherein the hemp oil extract is comprised at a content of 30-40 wt% based on the total weight of the formulation.

15. The intraoral fast disintegrating formulation according to any of claims 1 to 13, wherein the hemp powder extract is comprised at a content of 25-35 wt% based on the total weight of the formulation.

16. The intraoral fast disintegrating formulation according to any of claims 1 to 15, wherein the inorganic material is comprised at a content of 0.5-3 wt% based on the total weight of the formulation.

17. The intraoral fast disintegrating formulation according to any of claims 1 to 16, wherein, when the formulation comprises hemp oil extract, the hemp oil extract, the water-soluble polymer and the inorganic material are mixed at a weight ratio (hemp oil extract : water-soluble polymer : inorganic material, which is a water-insoluble excipient) of 7-12 : 8-12 : 0.1-3.

18. The intraoral fast disintegrating formulation according to any of claims 1 to 17, wherein, when the formulation comprises hemp powder extract, the hemp powder extract, the water-soluble polymer and the inorganic material are mixed at a weight ratio (hemp powder extract: water-soluble polymer : inorganic material) of 3-10 : 6-15 : 0.05-1.

19. The intraoral fast disintegrating formulation according to any of claims 1 to 18, wherein the formulation is an intraoral fast disintegrating film.
